Europäisches Patentamt

European Patent Office

Office européen des brevets

(11) Veröffentlichungsnummer: **0 173 919**
**B1**

(12) **EUROPÄISCHE PATENTSCHRIFT**

(45) Veröffentlichungstag der Patentschrift: **29.08.90**

(51) Int. Cl.⁵: **C 12 N 9/90, C 12 Q 1/54**

(21) Anmeldenummer: **85110549.4**

(22) Anmeldetag: **22.08.85**

(54) Verfahren zur mikrobiologischen Herstellung von Aldose-1-epimerase.

(30) Priorität: **05.09.84 DE 3432570**

(43) Veröffentlichungstag der Anmeldung:
**12.03.86 Patentblatt 86/11**

(45) Bekanntmachung des Hinweises auf die
Patenterteilung:
**29.08.90 Patentblatt 90/35**

(84) Benannte Vertragsstaaten:
**BE CH DE FR GB IT LI NL SE**

(56) Entgegenhaltungen:
FR-A-2 201 768

CHEMICAL ABSTRACTS, Band 68, 20. Mai 1968,
Seite 8972, Zusammenfassung Nr. 93109j,
Columbus, Ohio, US; W. SACKS: "Isolation and
properties of mutarotase in erythrocytes"

CHEMICAL ABSTRACTS, Band 98, 17. Januar
1983, Seite 414, Zusammenfassung Nr. 15489p,
Columbus, Ohio, US

(73) Patentinhaber: **MERCK PATENT GESELLSCHAFT
MIT BESCHRÄNKTER HAFTUNG
Frankfurter Strasse 250 Postfach 4119
D-6100 Darmstadt (DE)**

(72) Erfinder: **Ebeling, Wolfgang, Dr.
Am Hintergraben 9
D-6101 Bickenbach (DE)**
Erfinder: **Metz, Harald, Dr.
Am Landbach 8
D-6101 Bickenbach (DE)**
Erfinder: **Brümmer, Wolfgang, Dr.
Am Grenzweg 9
D-6146 Alsbach (DE)**
Erfinder: **Schmid, Günter, Dr.
Ebenstädterstrasse 18
D-6101 Mühltal (DE)**
Erfinder: **Behrendt, Ulrich, Dr.
Heimgartenstrasse 5
D-8177 Bischel/Obb. (DE)**

(56) Entgegenhaltungen:
CHEMICAL ABSTRACTS, Band 82, 23. Juni 1975,
Seite 250, Zusammenfassung Nr. 167297g,
Columbus, Ohio, US; P. SAMMLER et al.:
"Mutarotase in galactose-induced baker's yeast"

**EP 0 173 919 B1**

(56) Entgegenhaltungen:

CHEMICAL ABSTRACTS, Band 79, 17. September 1973, Seite 189, Zusammenfassung Nr. 63239m, Columbus, Ohio, US; S.A. MULHERN et al.: "Physical characteristics and chemiosmotic transformations of mutarotases from various species"

CHEMICAL ABSTRACTS, Band 76, 28. Februar 1972, Seite 162, Zusammenfassung Nr. 43405x, Columbus, Ohio, US; F. HUCHO et al.: "Enzymically catalyzed mutarotation. Mechanism of action of mutarotase (aldose 1-epimerase) from Escherichia coli"

CHEMICAL ABSTRACTS, Band 67, 20. November 1967, Seite 9127, Zusammenfassung Nr. 97129t, Columbus, Ohio, US; J.M. BAILEY et al.: "Mutarotases. I. Purification and properties of a mutarotase from higher plants"

# EP 0 173 919 B1

## Beschreibung

Die Erfindung betrifft ein Verfahren zur mikrobiologischen Herstellung von Aldose-1-epimerase (Mutarotase) durch Züchtung eines zur Bildung dieses Enzymes geeigneten Mikroorganismus der Spezies Acinetobactercalcoaceticus.

Mutarotase (EC 5.1.3.3) beschleunigt die Gleichgewichtseinstellung zwischen den $\alpha$- und $\beta$-Anomeren von Aldohexosen, z.B. zwischen $\alpha$- und $\beta$-Glucose, $\alpha$- und $\beta$-Galactose. Die Hauptanwendung des Enzyms liegt in der analytischen Biochemie bei der Beschleunigung enzymatischer Nachweisreaktionen für Aldosen mit Hilfe von für die $\alpha$-oder $\beta$-Form spezifischen Enzymen, bei denen die Gleichgewichtseinstellung zwischen beiden Anomeren der geschwindigkeitsbestimmende Schritt ist, z.B. bei Bestimmungsmethoden mit Glucosedehydrogenase. Glucoseoxidase, Galactosedehydrogenase und Galactoseoxidase. Eine großtechnische Anwendung könnte z.B. für den Glucoamylase/Glucoseisomerase-Prozeß interessant werden, weil Glucoamylase $\beta$-Glucose frei setzt, die erst nach Mutarotation zur $\alpha$-Form von der Glucoseisomerase umgesetzt werden kann.

Mutarotase ist in der Natur weit verbreitet. Sie kommt in verschiedenen Mikroorganismen (Bakterien, Hefen, Fadenpilzen), in Pflanzen und in tierischen Geweben vor.

Nennenswerte Enzymgehalte, die eine Isolierung von Mutarotase im technischen Maßstab ermöglichen, wurden bisher nur in Nieren von Säugetieren (Rind, Schwein) gefunden; alle bekannten Handelsprodukte werden aus Nieren hergestellt. Aus Bailey, Meth. Enzymol. 1975, S. 478, ist bekannt, daß Rindernieren mehr als die 60-fache Aktivität pro g Frischgewicht enthalten als z.B. Escherichia coli. In Biochim. Biophys. Acta *662*, 285 (1981) wird erstmals ein Verfahren zur mikrobiologischen Herstellung von Mutarotase aus Stämmen von Aspergillus niger beschrieben. Danach wurde aus dem besten Stamm eine Mutarotaseaktivität von 4,4 mU/ml Kulturbrühe erzielt, die Michaelis Konstante betrug 50 mM, das pH-Optimum lag im Bereich von 5—7.

Das bekannte mikrobiologische Verfahren hat jedoch eine sehr niedrige Ausbeute im Vergleich zu dem Herstellungsverfahren für Mutarotase aus Rindernieren. Außerdem sind die Charakteristika des Enzyms aus Aspergillus niger für die Gleichgewichtseinstellung bei enzymatischen Bestimmungen von Aldosen sehr ungünstig.

Der Erfindung liegt die Aufgabe zugrunde, ein Verfahren zur mikrobiologischen Herstellung von Aldose-1-epimerase (Mutarotase) zur Verfügung zu stellen bzw. das bekannte Verfahren zu verbessern, insbesondere hinsichtlich der Enzymausbeute. Das Verfahren sollte ausreichend hohe Aktivitäten liefern, möglichst vergleichbar mit denen aus Säugetiernieren; die gebildete Aktivität sollte in den Zellen möglichst lange und stabil erhalten blieben; das gebildete Enzym sollte günstige Eigenschaften hinsichtlich optimaler Substratkonzentration, Stabilität und pH-Optimum haben; störende Fremdaktivitäten oder andere Verunreinigungen in den Rohextrakten sollten möglichst gering sein oder sich leicht abtrennen lassen.

Gelöst wird diese Aufgabe erfindungsgemäß durch ein Verfahren zur mikrobiologischen Herstellung von Aldose-1-epimerase, das dadurch gekennzeichnet ist, daß man einen zur Bildung von Aldose-1-epimerase fähigen Mikroorganismus der Spezies Acinetobactercalcoaceticus in einem Nährmedium züchtet und nach Freisetzung aus den Zellen das Enzym abtrennt. Bevorzugte Mikroorganismen sind diejenigen der Stämme Acinetobacter calcoaceticus DSM 30 007, DSM 30 008, DSM 30 010 oder DSM 30 011 sowie deren Mutanten oder Varianten.

Im Vergleich zu der nach den bekannten Verfahren hergestellten Mutarotase ergeben sich außer der extremen Ausbeutesteigerung bis zu 1000 mU/ml Kulturbrühe eine Reihe weiterer Vorteile:

Bakterielles Ausgangsmaterial ist jederzeit in beliebigen Mengen herstellbar, so daß keine Probleme wie bei der Beschaffung und Lagerung großer Organmengen bestehen; die Anreicherung und Aufreinigung von Mutarotase aus Bakterienrohextrakten bis zu einem für diagnostische Zwecke anwendbaren Reinheitsgrad ist nach einfacheren, effektiveren und kostengünstigeren Verfahren möglich als bei der Gewinnung aus Organen. Wie die aus Rindernieren und aus Aspergillus niger gewonnene Mutarotase besitzt auch die erfindungsgemäß hergestellte Mutarotase eine hohe pH- und Temperaturstabilität und läßt sich problemlos lyophilisieren. Außerdem weist die erfindungsgemäß hergestellt Mutarotase jedoch einige charakteristische Vorteile auf, die sich günstig auf die Anwendung im Glucose-Bestimmungssystem auswirken: Gegenüber der Mutarotase aus Rindernieren ($K_M = 18$—20 mM) und der aus Aspergillus niger ($K_M = 50$ mM) hat die erfindungsgemäße Mutarotase eine um den Faktor 3 bzw. 8 kleinere Michaelis-Konstante ($K_M = 6$—8 mM). Gerade im suboptimalen Substratbereich, in dem man bei einer Glucose-bestimmung mißt, wirkt sich die größere Affinität des bakteriellen Enzyms positiv aus. Gegenüber Galactose weist die Mutarotase aus Acinetobacter eine 3-fach höhere Michaelis-Konstante auf als die Mutarotase aus Rindernieren, d.h. im suboptimalen Bereich wird die Aktivität der Mutarotase aus Acinobacter durch Galactose in Bezug auf Glucose weniger gestört als beim Enzym aus Rindernieren.

Die Mutarotase aus Acinetobacter hat gegenüber derjenigen aus Rindernieren und aus Aspergillus niger ein pH-Optimum, das etwas stärker im alkalischen Bereich liegt (pH 7—8). Dies hat zu Folge, daß bei einer Glucose-Bestimmung (pH 7,6) die pH-Optima der Glucosedehydrogenase und der Mutarotase noch besser aufeinander abgestimmt sind und sich hervorragend ergänzen. Die Mutarotase aus Acinetobacter zeigt eine noch weitergehendere Unabhängigkeit von SH-Schutzreagenzien als die Mutarotase aus Rindernieren. Da die Glucosedehydrogenase keinen SH-Schutz benötigt, ergänzen sich auch in diesem

3

EP 0 173 919 B1

Punkt die beiden zur Glucosebestimmung eingesetzten Enzyme in außergewöhnlicher Weise.

Als stark basisches Protein bindet die erfindungsgemäße Mutarotase gut an Kationenaustauscher, was zu einer verbesserten und einfacheren Reinigung führt. Das nach diesem Verfahren gewonnene Produkt enthält weniger störende Begleitsubstanzen wie Pigmente, enzymatische Fremdaktivitäten (LDH, NADH-Oxidase aktivierende Substanzen) und Substanzen, die bei der späteren Verwendung der Mutarotase als Bestandteil von Reagenzlösungen zu Trübungen und Ausflockung führen können.

Die Mikroorganismen werden als Schrägagarkulturen auf üblichen Nährmedien, die z.B. Fleischextrakt, Peptone und/oder Hefeextract und/oder anorganische Salze und zusätzliche C-Quellen enthalten, angezüchtet. Zur weiteren Vermehrung der Organismen können im Submersverfahren Schüttel- oder Standkulturen in entsprechenden Nährlösungen angezüchtet werden. Die Anzucht der Kulturen erfolgt zwischen 20 und 40°C, vorzugsweise bei 25—36°C. Ausgehend von einer gut gewachsenen, etwa 5 bis 36 Stunden alten Kultur können Fermenter beimpft werden, welche z.B. eine Nährlösung auf Basis Hefeextrakt, Cornsteep, Pepton und/oder Mineralsalzen und gegebenenfalls einer zusätzlichen Kohlenstoff-Quelle enthalten können. Als zusätzliche Kohlenstoff-Quellen eignen sich Carbonsäuren wie Milchsäure, Citronensäure, Essigsäure oder auch Glucose, Fructose, Saccharose, Galactose, Xylose, Glycerin, 2,3-Butandiol, Ethanol, vorzugsweise Milchsäure und/oder Citronensäure.

Der Start-pH der Kultur sollte vorzugsweise zwischen 6,0 und 8,0 liegen. Zum Beimpfen der Nährlösung können Impfmengen von 0,1%—10% des Kulturvolumens verwendet werden. Die Züchtung kann sowohl im batch-Verfahren als auch teil- oder vollkontinuierlich in einer oder mehreren Stufen erfolgen.

Nach Erreichen das Aktivatätsmaximums werden die Zellen durch Zentrifugation oder durch andere geeignete Methoden abgetrennt und die Enzym-enthaltende Phase anschließend eingekühlt. Die Freisetzung der intrazellulären Mutarotase erfolgt bei kleinen Bakterienmengen durch Lysozymaufschluß, bei größeren Arbeitsvolumina durch mechanischen Zellaufschluß, beispielsweise mit einem Hochdruckhomogenisator, mit Glasperlenmühlen, durch osmotische Behandlung der Zellen oder durch andere geeignete Verfahren zur Freisetzung von intrazellulär gebildeten bakteriellen Enzymen. Für technische Zwecke können auch die erhaltenen Zellen anstelle des freigesetzten Enzyms verwendet werden.

Der Bakterienschlamm wird in der Regel unter Kühlung und in Gegenwart eines Detergenz durch Hochdruckbehandlung mit Hilfe eines Hochdruckhomogenisators aufgeschlossen. Die Bakterientrümmer werden so weit wie möglich durch Zentrifugieren oder andere geeignete Methoden abgetrennt. Aus dem trüben Überstand können Begleitsubstanzen durch Fällungsmittel wie Ammoniumsulfat oder Polyethylenglycol präzipitiert werden. Das Enzym läßt sich nach einer anschließenden Dialyse direkt an einen Kationenaustauscher adsorbieren. Die Dialyse der trüben Enzymlösung erfolgt vorzugsweise durch Ultrafiltration mit Röhrenmembranmodulen. Das Enzym wird in schwach saurem Milieu an den Ionenaustauscher adsorbiert, der in trockner Form in die Enzymlösung eingetragen wird. Der beladene Austauscher wird auf der Nutsche gewaschen bis das Waschwasser klar ist. Danach wird er in eine Chromatographiesäule gepackt und die Mutarotase mit einem Kaliumchlorid-Gradienten eluiert und anschließend lyophilisiert.

Nach dem erfindungsgemäßen Verfahren läßt sich eine Mutarotase isolieren, die durch folgende Eigenschaften charakterisiert ist: das Molekulargewicht beträgt etwa 80 000 d (4 Untereinheiten a 20 000 d), das pH-Aktivitätsoptimum liegt im pH-Bereich von 7 bis 8, das Temperaturoptimum bei 42°C bis 46°C. Die Michaelis-Konstante für Glucose beträgt 6—8 mM.

Über einen Zeitraum von einem halben Jahr war die Aktivität der Mutarotase sowohl bei 4°C, 25°C als auch bei 30°C und pH-Werten von 6,7—8,4 stabil. Eine Lyophilisation ist ohne nennenswerte Aktivatätsverluste problemlos möglich.

Die Aktivität der erfindungsgemäß hergestellten Mutarotase ist abhängig von der Anwesenheit freier Sulfhydryl-Gruppen, die durch SH-Reagenzien wie N-Ethylmaleinimid, 5,5'-Dithio-bis-(2-nitrobenzoesäure) und Jodacetat blockiert werden können. Ein besonderer SH-Schutz in Verdünnungs- und Testpuffern ist jedoch nicht erforderlich. Bei Konzentrationen von 2 mM hemmen $Hg^{++}$-, $Fe^{+++}$-Ionen die Aktivität etwa zur Hälfte; $Cu^{++}$-, $Co^{+++}$- und $Zn^{++}$-Ionen hemmen in gleicher Konzentration vollständig.

Die Mutarotase ist spezifisch für Aldohexosen. Die Umsatzgeschwindigkeit für Galactose ist 3,5-fach höher als für Glucose bei gleicher Michaelis-Konstante. Die Michaelis-Konstante für Glucose liegt bei 6—8 mM.

Die Aktivitätsbestimmung wird wie folgt durchgeführt:

*Prinzip:*

$$\alpha\text{-Glucose} \quad \overset{\text{Mutarotase}}{\rightleftharpoons} \quad \beta\text{-Glucose}$$

$$\beta\text{-Glucose} + NAD \quad \xrightarrow{\text{Glucosedehydrogenase}} \quad Gluconolacton + NADH_2$$

Man läßt Mutarotase, Glucosedehydrogenase und NAD mit einer frisch angesetzten Lösung von $\alpha$-Glucose reagieren und bestimmt das gebildete $NADH_2$ durch Extinktionsmessung bei 366 nm. Die

4

Messung der Beschleunigung der NADH$_2$-Bildung durch Mutarotase (verglichen mit einem Blindwert ohne Mutarotase) gestattet die Berechnung der Mutarotase-Aktivität in der eingesetzten Lösung.

Testansatz:
1. 0,2 M Trispuffer pH 7,2.
2. Glucosedehydrogenase, 75 U/ml in 3 M Kochsalzlösung.
3. NAD, 40 mg/ml bidest. Wasser.
4. α-Glucose, 30 mg in 100 ml dest. Wasser.

Alle Reagenzien werden auf 25°C temperiert; das dest. Wasser zum Lösen der α-Glucose wird auf 20°C temperiert.

|  | Analyse | Blindwert |
|---|---|---|
| Lösung 1 | 1,00 ml | 1,00 ml |
| Lösung 2 | 0,10 ml | 0,05 ml |
| Lösung 3 | 0,05 ml | 0,05 ml |
| Mutarotase-Lösung | 0,10 ml | — |
| Bidest. H$_2$O | — | 0,10 ml |

Sobald die Extinktion konstant ist, wird gestartet mit je 0,10 ml der Lösung 4.

Für jede Messung wird eine frische Glucoselösung angesetzt. Zur Erfassung der Eigenmutarotation ist folgender Arbeitsgang unbedingt einzuhalten: 100 ml dest. Wasser werden in ein 150 ml-Becherglas gefüllt und auf 20°C temperiert. Das Becherglas wird gleichmäßig gerührt, 30 mg α-Glucose werden zugegeben, die Stoppuhr gedrückt und der Zeitpunkt am Schreiberprotokoll markiert. Es wird 30 Sekunden gemischt. Innerhalb der nächsten 15 Sek. wird die Glucoselösung in die Küvette pipettiert, der Küvetteninhalt wird gut durchmischt und nach Ablauf dieser 15 Sek. die Zunahme der Extinktion registriert. Die Messung erfolgt bei 366 nm, Schichtdicke 1 cm, Küvettentemperierung 25°C.

Zur Auswertung wird eine Tangente an den linearen Teil am Anfang der Registrierkurve angelegt und $\Delta E$/min. abgelesen; die Subtraktion des Blindwertes vom Analysenwert ergibt den Wert $\Delta\Delta E$/min., welcher in folgende Berechnungsformel eingesetzt wird:

Enzymaktivität = $\Delta\Delta E$/min. × 4,09 U Mutarotase pro ml eingesetzte Enzymlösung

Rohe Mutarotase-Proben sind auf Glucosefreiheit zu prüfen, da der eingebrachte β-Glucose-Anteil den Test stört.

Beispiel 1

In einem Fermenter werden 100 Liter einer sterilen Nährlösung folgender Zusammensetzung

| | |
|---|---|
| Pepton aus Casein | 0,5 % |
| Hefeextrakt | 1,0 % |
| Cornsteep-Pulver | 0,3 % |
| Dikaliumhydrogenphosphat | 0,8 % |
| Magnesiumsulfatheptahydrat | 0,04 % |
| Glucose | 1,2 % |
| Silicon-Entschäumer | 20 ml |
| pH-Wert | 6,8 |

mit 1 l einer 18 Stunden alten Submerskultur von Acinetobacter calcoaceticus M 30 007 beimpft der in der gleichen Nährlösung angezüchtet wurde.

Diese Kultur wird 18 Studen bei mittlerer Belüftung (Rührer: 250 Upm, Luft: 60 l/min, Überdruck: 0,5 bar) und 34°C bebrütet.

Während der ersten 2—10 Studen nimmt die Zellmase, gemessen als Trübung, zu und bleibt dann konstant. Nach 10 Stunden hat die Kultur ihr Aktivitätsmaximum der Mutarotase mit 340 U/l erreicht.

EP 0 173 919 B1

Die Freisetzung der Mutarotase aus den Bakterien und die Aktivitätsbestimmung erfolgen nach folgendem Verfahren.

3 ml einer gut durchmischten, glucosefreien Fermenterprobe werden 10 Min. bei 5000 U/min. abzentrifugiert und der Überstand verworfen. Die Bakterienmasse wird in 3 ml Phosphat-Puffer 0,1 pH 6,5 und 0,1 ml EDTA-Lösung (1,8 g/l) suspendiert und im Zentrifugenröhrchen im Aceton-Trockeneis-Kältebad schnell eingefroren (ca. 15 Min.) und anschließend im ca. 40°C Wasserbad aufgetaut. 1 Tropfen eines Detergenz und 25—30 mg Lysozym werden zugegeben (ca. 15 000 U/mg) und bei 28°C mit dem Magnetrührer 15 Min. lang gerührt. Anschließend wird zur Inaktivierung von NADH-Oxidasen 5 Min. lang auf 45°C erwarmt, aufgeschlossen und mit einer Zentrifuge klar geschleudert (ca. 2 Min.). Die Enzymbestimmung erfolgt im klaren Überstand.

Beispiel 2

Enzymaufarbeitung

100 l Bakterienschlamm werden mit 0,5% eines Detergenz und 0,1 Mol/l Kaliumchlorid versetzt. Die Zellen werden mit einem Hochdruckhomogenisator bei 500 bar aufgeschlossen. Der Bakterienschlamm muß dabei gekühlt werden. Der Zelltrümmer werden mit einer Zentrifuge (3000 × g; Laufzeit 3 Stunden) so weit wie möglich abgetrennt. Der Rückstand wird verworfen, das trübe Zentrifugat wird mit 20% Polyethylenglycol versetzt, um Begleitsubstanzen auszufällen. Man rührt 30 Minuten nach und zentrifugiert den Niederschlag ab. Der immer noch trübe Üerstand enthält etwa 2 U/ml Mutarotase und wird gegen Wasser diafiltriert bis zu einer Leitfähigkeit von $3 \times 10^{-3}$ S. Nach der Diafiltration wird die Mutarotase bei pH 5,5 im batch-Verfahren an einen trocknen Ionenaustauscher adsorbiert. Für 20000 U sind 5—10 g Austauscher erforderlich. Der beladene Austauscher wird abgesaugt und solange mit 0,025 M Kaliumphosphatpuffer, pH 5,5, gewaschen, bis das Filtrat frei von Trübung ist. Danach wird der beladene Austauscher in eine Säule gepackt, mit 0,025 M Kaliumphosphatpuffer, pH 5,5, extinktionsfrei gewaschen und anschließend mit einem Kaliumchlorid-Gradienten eluiert.

Die enzymhaltigen Fraktionen werden gesammelt, durch Ultrafiltration auf 60—100 U/ml konzentriert und anschließend lyophilisiert. Die Ausbeute beträgt 70%, die spezifische Aktivität etwa 6,5 U/mg Protein.

Beispiel 3

Als Stamm wird Acinetobacter calcoaceticus M 30 008 eingesetzt. Kulturführung und Nährlösung entsprechen Beispiel 1 mit der Ausnahme, daß nur 1% Glucose vorgelegt wurde, während anschließend 3,5% Glucose kontinuierlich zudosiert wurden. Nach 15 Stunden hat die Kultur ihr Aktivitätsmaximum der Mutarotase mit 490 U/l erreicht.

Beispiel 4

Als Stamm wird Acinetobacter calcoaceticus M 30 010 verwendet. Die Kulturführung und die Nährlösung entsprechen Beispiel 3, jedoch wird Glucose durch 1% Natriumlactat ersetzt. Während des Wachstums wird 20 %iger Milchsäure pH-Wert abhängig zutitriert oder mit konstanter Rate zudosiert und der pH-Wert mit Ammoniak auf pH 6,8 reguliert. Nach 12 Stunden hat die Kultur ihr Mutarotase-Aktivitätsmaximum von 1200 U/l erreicht.

Beispiel 5

Die Züchtung erfolgt analog Beispiel 4, jedoch wird statt Milchsäure 50 %iger Citronensäure verwendet. Nach 10 Stunden wird die Kulturführung in Gegenwart eines anionischen, kationischen oder nichtionischen Tensids, vorzugsweise N-Cetylpyridiniumchlorid in einer Konzentration von 0,01 bis 0,1% vorgenommen. Das Mutarotase-Aktivitätsmaximum von 2200 U/l wird nach 20 Stunden erreicht.

**Patentansprüche**

1. Verfahren zur mikrobiologischen Herstellung von Aldose-1-epimerase durch Züchtung von Mikroorganismen in einem Nährmedium und Freisetzung des Enzyms aus den Zellen, dadurch gekennzeichnet, daß man als Mikroorganismus Acinetobacter calcoaceticus verwendet.

2. Verfahren nach Anspruch 1, dadurch gekennzeichnet, daß man als Mikroorganismus einen der Stämme Acinetobacter calcoaceticus DSM 30 007, DSM 30 008, DSM 30 010 oder DSM 30 011 verwendet.

3. Verfahren nach den Ansprüchen 1 und 2, dadurch gekennzeichnet, daß man in einem Nährmedium züchtet, das als zusätzliche Kohlenstoffquelle eine Carbonsäure enthält.

4. Verfahren nach den Ansprüchen 1 bis 3, dadurch gekennzeichnet, daß man in einem Nährmedium züchtet, das als zusätzliche Kohlenstoffquelle Milchsäure und/oder Citronensäure enthält.

5. Aldose-1-epimerase erhältlich nach dem Verfahren gemäß den Ansprüchen 1—4 und charakterisiert durch die folgenden Eigenschaften:

(a) Molekulargewicht: etwa 80 000 d (4 Untereinheiten á 20 000 d)

(b) pH-Aktivitätsoptimum: pH 7—8

(c) Temperaturoptimum: 42°C—46°C

(d) Michaelis-Konstante: 6—8 mM für Glucose

6. Verwendung von Aldose-1-epimerase nach den Ansprüchen 1 bis 5 in Aldosebestimmungssystemen mit Glucosedehydrogenase, Glucoseoxidase, Galactosedehydrogenase, Galactoseoxidase.

6

# EP 0 173 919 B1

**Revendications**

1. Procédé pour la production microbiologique d'aldose-1-épimérase par culture de micro-organismes dans un milieu nutritif et extraction de l'enzyme des cellules, caractérisé en ce que l'on utilise comme micro-organisme l'Acinetobacter calcoaceticus.

2. Procédé selon la revendication 1, caractérisé en ce que l'on utilise comme micro-organisme l'une des souches Acinetobacter calcoaceticus DSM 30 007, DSM 30 008, DSM 30 010 ou DSM 30 011.

3. Procédé selon les revendications 1 et 2, caractérisé en ce que l'on réalise la culture dans un milieu nutritif contenant un acide carboxylique comme source de carbone supplémentaire.

4. Procédé selon les revendications 1 à 3, caractérisé en ce que l'on réalise la culture dans un milieu nutritif contenant de l'acide lactique et/ou de l'acide citrique comme source de carbone suppélementaire.

5. Aldose-1-épimérase obtenue suivant le procédé décrit aux revendications 1 à 4 et caractérisée par les propriétés suivantes:
(a) poids moléculaire: environ 80 000 d (4 sous-unités de 20 000 d)
(b) pH d'optimum d'activité: pH 7—8
(c) optimum de température: 42°C—46°C
(d) constante de Michaelis: 6—8 mM pour le glucose.

6. Utilisation de l'aldose-1-épimérase selon les revendications 1 à 5 dans les systèmes de détermination des aldoses avec la glucose-déhydrogénasè, la glucose-oxydase, la galactose déhydrogénase et la galactose-oxydase.

**Claims**

1. Process for the microbiological preparation of aldose-1-epimerase by cultivating microorganisms in a nutrient medium and releasing the enzyme from the cells, characterized in that Acinetobacter calcoaceticus is used as the microorganism.

2. Process according to Claim 1, characterized in that one of the strains Acinetobacter calcoaceticus DSM 30,007, DSM 30,008, DSM 30,010 or DSM 30,011 is used as the microorganism.

3. Process according to Claims 1 and 2, characterized in that the cultivation is carried out in a nutrient medium which contains a carboxylic acid as an additional carbon source.

4. Process according to Claims 1 to 3, characterized in that the cultivation is carried out in a nutrient medium which contains lactic acid and/or citric acid as an additional carbon source.

5. Aldose-1-epimerase obtainable by the process according to Claims 1 to 4 and characterized by the following properties:
(a) molecular weight: about 80,000 d (4 subunits of 20,000 d each)
(b) pH activity optimum: pH 7—8
(c) temperature optimum: 42°C—46°C
(d) Michaelis constant: 6—8 mM for glucose.

6. Use of aldose-1-epimerase according to Claims 1 to 5 in aldose determination systems with glucosedehydrogenase, glucose-oxidase, galactose-dehydrogenase and galactose-oxidase.